(19)

Europäisches
Patentamt
European
Patent Office
Office européen
des brevets

(11) **EP 2 570 488 A1**

(12) **EUROPEAN PATENT APPLICATION**

(43) Date of publication:
**20.03.2013 Bulletin 2013/12**

(51) Int Cl.:
*C12Q 1/68* *(2006.01)* *B01L 3/00* *(2006.01)*

(21) Application number: **11306169.1**

(22) Date of filing: **16.09.2011**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicant: **Centre National de la Recherche
Scientifique
(C.N.R.S)
75016 Paris (FR)**

(72) Inventors:
• **Bancaud, Aurélien
31400 Toulouse (FR)**
• **He, Qihao
31400 Toulouse (FR)**

(74) Representative: **Colombet, Alain André et al
Cabinet Lavoix
62, rue de Bonnel
69448 Lyon Cedex 03 (FR)**

(54) **Method for longitudinal macromolecule spreading and method for analyzing macromolecules**

(57)     The present invention relates to a method for longitudinal macromolecule spreading by passing a fluid comprising said macromolecule in a nanochannel by applying to this fluid a hydrostatic pressure.

The invention also relates to a method for analyzing at least one macromolecule.

**EP 2 570 488 A1**

**Description**

The present invention concerns a method for longitudinal macromolecule spreading. The invention also relates to the use of said method to monitor the conformation of a macromolecule in an elongated state for analysis, especially structural analysis. The invention also relates to a method for analyzing macromolecules, especially for analyzing the molecular constitution of macromolecules, especially the genomic or epigenomic information they are containing, for example for the diagnosis of diseases such as cancer.

Generally, the macromolecules, for examples DNA, adopt, for energy minimization reasons, a non-linear globular 3-D conformation. Because of this folded conformation, for example in case of DNA, it is difficult to retrieve the genomic or epigenomic information. It is thus necessary to linearize said macromolecule for analyzing its structure.

Different techniques have been disclosed for macromolecule spreading into a linear conformation. For example, DNA molecular combing techniques are known. They consist of spreading out DNA on a surface by working on hydrophilic/hydrophobic interactions between said surface and DNA. However, those techniques do not enable to precisely monitor the regions where macromolecules are extended in a linear conformation and the process suffers from an intrinsic lack of reproducibility associated to the unreliable surface modification protocols.

Austin et al have carried out some experiments on macromolecule spreading using nanotechniques (US7670770 and US7217562). Those techniques are based on an entropic spreading of a macomolecule when it is physically confined in a nanochannel presenting a rectangular or square section presenting a width of less than 150 nm, a height of less than 200 nm and a length greater than 1 $\mu$m. However, it is difficult to enter a macromolecule into a nanochannel, and the macromolecule generally enters the nanochannel in a hairpin conformation which is not satisfactory for linear analysis. Austin et al thus developed techniques based on a network of obstacles the macromolecule has to traverse before entering the nanochannel, the obstacle network enabling a pre-spreading of the macromolecule before its entrance into the nanochannel. Once in the nanochannel, the degree of spreading of the molecule is controlled by the confinement induced by the channels walls, so those techniques require channels of very low dimensions to reach sufficient spreading of the macromolecules.

It is therefore necessary to provide an improved method for macromolecule spreading which does not require a high containment of the macromolecule.

The objective of the present invention is to provide a method for improved macromolecule spreading compared to the methods of the prior art.

The objective of the present invention is also to provide a method for macromolecule spreading which is easier to carry out compared to the methods of the prior art.

Another objective of the present invention is also to provide a method for analyzing macromolecules, especially for analyzing the constitutive genomic or epigenomic information of biological macromolecules.

Other objectives will appear by reading the following description of the invention.

The invention relates to a method for longitudinal macromolecule spreading by passing a liquid comprising said macromolecule in a nanochannel by applying to this liquid a hydrostatic pressure. This results in the macromolecule passing through the nanochannel in a spreaded conformation. The spreaded macromolecule may be subjected to analysis during its passage through the nanochannel and/or after or at the time the macromolecule goes out the nanochannel.

The macromolecule may be natural, synthetic or artificial. It is especially an elongated macromolecule, preferably a biomacromolecule which may include nucleic acids, such as RNA, DNA, single-stranded or double-stranded, peptide nucleic acids (PNA), unfolded proteins, polypeptides or peptide chains, polymer chains or copolymer chains, chromosomes or chromosome fragments.

The present invention is particularly suited for nucleic acids and especially double-stranded DNA.

According to the invention, a macromolecule is a molecule of molecular mass greater than or equal to 100 kDa, preferably comprised between $10^5$ and $10^{10}$ Da, for example between $10^7$ and $10^9$ Da. The persistence length of macromolecules, which is defined as the length over which correlations in the direction of the tangent are lost due to thermal fluctuations, is preferably comprised between 1 and 200 nm, preferably comprised between 20 and 100 nm.

The persistence length of a macromolecule may be measured by methods well known by the skilled person, for example by electronic microscopy by following the evolution of its conformation, by analyzing the tortuosities of its contour, or by measuring the hydrodynamic diameter of the macromolecule which, using a first order approximation that neglects intrachain molecular repulsion, is calculated as follows: persistence length x (number of monomer)$^{1/2}$.

For example, for double-stranded DNA, the persistence length is about 50 nm, for single-stranded DNA, it is about 3 nm and for a chromosome it is comprised between 30 and 100 nm.

The present invention is well suited for macromolecules which length is at least 10 times greater than its persistence length, or even 100 times greater than its persistence length.

According to the invention "macromolecule spreading" means a spreading of the macromolecule to give it a more linear conformation compared to its natural conformation, especially a non-linear 3-D globular conformation. The degree of spreading represents the percentage of the length of linear portion of the macromolecule (in $\mu$m) compared to its contour length (or its theoretical total length) if it was completely linear. According to the present invention "spreading" and "stretching" are equivalent terms.

Preferably, the method is carried out in a steady state.

The nanochannel can present a section characterized by a larger diagonal (or diameter if circular) is comprised advantageously between 100 nm and 600 nm, preferably between 200 nm and 300 nm, for example between 200 nm and 250 nm.

The nanochannel can present a rectangular, square, circular or oval section. This section can be characterized by a larger diagonal (or diameter if circular) is comprised advantageously between 100 nm and 600 nm, preferably between 200 nm and 300 nm, for example between 200 nm and 250 nm.

Preferably, the nanochannel has a length (L) which is greater than or equal to the theoretical total length of the macromolecule. In an embodiment, the nanochannel has a length (L) greater or equal to 20 $\mu$m, preferably comprised between 20 and 1000 $\mu$m, more preferably comprised between 100 and 200 $\mu$m, for example comprised between 100 and 150 $\mu$m. This length is preferably selected to be greater than or equal to the theoretical total length of the macromolecule.

In an embodiment, the nanochannel may present a circular section with a diameter comprised between 150 and 300 nm, preferably between 200 and 250 nm. This nanochannel may further have a length (L) greater or equal to 20 $\mu$m, preferably comprised between 20 and 10 000 $\mu$m, more preferably comprised between 20 and 1000 $\mu$m, for example comprised between 100 and 200 $\mu$m.

In another embodiment the nanochannel presents a rectangular or square section, preferably with:

- a width (W) comprised between 100 and 500 nm, preferably between 150 and 300 nm, more preferably between 200 and 300 nm;
- a height (H) comprised between 100 and 500 nm, preferably between 150 and 300 nm, more preferably between 200 and 300 nm; and
- a length (L) comprised between 100 and 200 $\mu$m, preferably between 100 and 150 $\mu$m.

According to the method of the invention, a hydrostatic pressure is applied to the fluid comprising the macromolecule which enables the advection of said fluid in the nanochannel.

The hydrostatic pressure applied to the fluid, and thus the pressure at the inlet of the nanochannel may be constant.

When several nanochannels are implemented, the pressure at the inlet of each nanochannel could be the same or different.

Preferably, the pressure at the inlet of the nanochannel(s) is less than 1 MPa, preferably less than 0.2 MPa, for example comprised between 0.01 and 0.7 MPa, in particular comprised between 0.01 and 0.2 MPa, for example between 0.01 and 0.1 MPa.

The pressure at the outlet of the nanochannel(s) is less than the pressure at the inlet of the nanochannel and can be the atmospheric pressure or a pressure greater than the atmospheric pressure, preferably comprised between 0.001 and 0.005 MPa, in particular comprised between 0.001 and 0.002 MPa, for example the pressure at the outlet of the nanochannel is of 0.001 MPa.

Specific means are employed to control the pressure applied to the fluid and thus the pressure at the inlet and at the outlet of the nanochannel in order to produce a suitable hydrostatic pressure. A suitable way is to use a system with two fluids, one fluid which is the one containing the macromolecule, the other is the actuating fluid which exerts the suitable pressure to the first. Both fluids may be liquids, with an appropriate interface between them to avoid direct contact. Preferably, the actuating fluid is a gas which can be in direct contact with the liquid. For example, the inlet of the nanochannel is connected to a first chamber which is the reservoir for the liquid containing the macromolecule. This chamber is pressurized in a controlled manner by the actuating fluid, preferably a gas which enters in direct contact with the liquid in the first chamber. The outlet of the nanochannel is connected to a second chamber which is at the atmospheric pressure or at another pre-determined or controlled pressure and enables to control the pressure at the outlet of the nanochannel. Suitable devices of this type are described in WO2004/103566.

The pressure drop in the nanochannel results from the pressure loss and possibly from the pressure at the outlet of the nanochannel. During the process of the present invention, a pressure drop is induced inside the nanochannel. The relation between the pressure drop in the nanochannel and the volumetric rate of the flow of liquid can be defined by the following equation:

$$\frac{\Delta P}{L} = \frac{a\mu}{WH^3}Q$$

wherein

$$a = 12\left(1 - \frac{192}{\pi^5} \times \frac{H}{W}\right)^{-1}$$

$\mu$ is the viscosity of the fluid and Q its volumetric flow rate. When the nanochannel has a circular section of radius R the relationship between the volumetric flow rate and the pressure drop as follows:

$$\frac{\Delta P}{L} = \frac{8\mu}{\pi R^4}Q$$

The value of the pressure drop to be applied mainly depends on the dimensions of the nanochannel and on the degree of macromolecule spreading wanted. Especially, for a same spreading degree, the pressure drop applied should be enhanced when the length of the nanochannel is enhanced. The volumetric flow rate is directly related to the mean fluid velocity inside nanochannels according to:

$$v_{mean} = \frac{Q}{S}$$

with S the surface of the nanochannel, and S=$\pi R^2$ or WH for cylindrical or rectangular nanochannels, respectively.

The skilled person could determine the dimensions of the nanochannel for a given pressure drop. For example:

- for a length comprised between 50 and 200$\mu$m, and a larger diagonal (or diameter if circular) comprised between 200 and 300 nm, the pressure drop between the inlet and outlet channels should be comprised between 0.002 and 0.016 MPa for a mean flow velocity of 100 $\mu$m/s ; or
- for a length comprised between 50 and 200 $\mu$m and a larger diagonal (or diameter if circular) comprised between 200 and 300 nm, the pressure drop should be comprised between 0.02 and 0.16 MPa for a mean flow velocity of 1000 $\mu$m/s.
   In case of nanochannel having a rectangular or a square section,
- for a length comprised between 50 and 200 $\mu$m, a width comprised between 200 and 300 nm, and a height comprised between 200 and 300 nm, the pressure drop between the inlet and outlet channels should be comprised between 0.002 and 0.016 MPa for a mean flow velocity of 100 $\mu$m/s ; or
- for a length comprised between 50 and 200 $\mu$m, a width comprised between 200 and 300 nm, and a height comprised between 200 and 300 nm, the pressure drop should be comprised between 0.02 and 0.16 MPa for a mean flow velocity of 1000 $\mu$m/s.

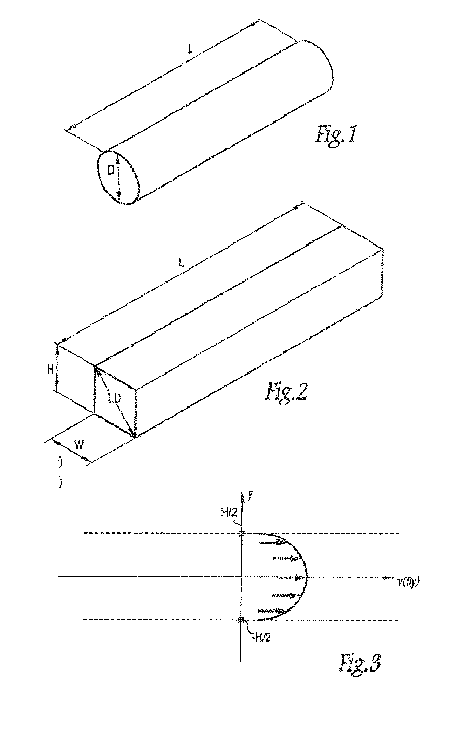

It should be understood that when starting the method of the invention, the nanochannel is filled with the fluid. Then, the implementation of the method according to the invention enables a flow of the fluid and the macromolecule in the nanochannel. Without being bound by the theory it is deemed that the hydrostatic pressure applied to the fluid, and thus the pressure at the inlet of the nanochannel induces a hydrodynamic flow into the nanochannel, this hydrodynamic flow forces the passage of the macromolecule into the nanochannel. Advantageously, this hydrodynamic flow presents a parabolic profile. Taking into account a 2D flow according to figure 2, the parabolic flow rate in the nanochannel can be related to the pressure drop as follows:

$$v(y) = \frac{\Delta P}{2\mu L} \times \left\{ \frac{H^2}{4} - y^2 \right\}$$

wherein y is comprised between -H/2 and H/2, H being the height of the nanochannel or the diameter when the nanochannel has a circular section. This expression is a good approximation of the fluid flow, given that H<W. Note that the mean flow velocity can then derived as follows:

$$v_{mean} = \frac{\Delta P}{2\mu L} \times \frac{H^2}{6}$$

As a consequence of this parabolic profile the flow rate is minimal at the vicinity of the nanochannel walls and maximal at its center. Because of the parabolic profile of the flow, the portions of the macromolecule which are near the center of the nanochannel move with a greater rate than the portions of the macromolecule which are near the walls of the nanochannel. This induces a shear stress of the macromolecule. Compression and elongation forces are thus exerted over the full molecular length. The effect of elongation forces, which tend to reduce the effect of confinement on the macromolecule, is favoured over compression forces that would tend to spread the molecule transverse to the nanochannel. Thus, in addition to the entropic spreading of the macromolecule due to its containment in the nanochannel, the macromolecule undergoes shear stresses that tend to increase its degree of stretching. A high spreading of the macromolecule can thus be obtained without using nanochannels of high confinement, which are challenging to produce. Overall, it is described herein a flow-driven spreading of the molecule, that allows to reach steady high degrees of elongation without strong confinements.

Advantageously, the hydrodynamic flow also induces a relaxation effect on the macromolecule and enables it not to adopt a hairpin conformation when entering the nanochannel.

Advantageously, the mean flow velocity is comprised between 10 and 10 000 $\mu$m/s, preferably between 100 and 1000 $\mu$m/s, for example between 100 $\mu$m/s and 500 $\mu$m/s. The value of the flow velocity mainly depends on the dimensions of the nanochannel and on the degree of macromolecule spreading wanted. The skilled person could determine the dimensions of the nanochannel for a given flow rate. Embodiments are:

- for a larger diagonal (or diameter if circular) comprised between 200 and 300 nm, the mean flow velocity ($v_{mean}$) is comprised between 50 and 500 $\mu$m/s; or
- for a larger diagonal (or diameter if circular) comprised between 200 and 400 nm, the mean flow velocity ($v_{mean}$) is comprised between 100 and 500 $\mu$m/s; or
- for a larger diagonal (or diameter if circular) comprised between 100 nm and 300 nm, the mean flow velocity ($v_{mean}$) is comprised between 50 and 200 $\mu$m/s.

In case of nanochannel having a rectangular or a square section, embodiments are:

- for a width comprised between 200 and 300 nm, and a height comprised between 200 and 300 nm, the mean flow velocity ($v_{mean}$) is comprised between 50 and 500 $\mu$m/s; or
- for a width comprised between 300 and 400 nm, and a height comprised between 200 and 300 nm, the mean flow velocity ($V_{mean}$) is comprised between 100 and 500 $\mu$m/s; or
- for a width comprised between 100 and 200 nm, and a height comprised between 200 nm and 300 nm, the mean flow velocity ($V_{mean}$) is comprised between 50 and 200 $\mu$m/s.

Advantageously, the method according to the invention enables a spreading of more than 30% of the total theoretical linear length of the macromolecule, preferably greater than or equal 60 % of the total theoretical linear length of the macromolecule, for example comprised between 40 and 90% of the total theoretical linear length of the macromolecule.

The presence of salts may modify the persistence length of the macromolecule. Advantageously, low salt concentrations enable to enhance the persistence length of the macromolecule.

Preferably, the fluid comprising the macromolecule is a buffer solution at physiological pH between 5 and 9, more preferably between 7 and 8. Preferably, the buffer solution is a TBE buffer solution, phosphate buffer solution, or carbonate buffer solutions, for example a TBE buffer solution from 0.01X to 1X. Preferably, the salt concentration in the fluid is comprised between 1 and 500 mM, more preferably between 50 and 200 mM.

Preferably, the concentration of the macromolecule in the fluid is comprised between 0.01 and 100 $\mu$M, more preferably between 0.1 and 10 $\mu$M.

According to another embodiment, the process according to the invention further comprises the implementation of an electric field to the fluid subjected to the hydrodynamic process of the invention. In an embodiment, the electric field is comprised between 1 and $10^4$ kV/m. There is thus a combination of hydrodynamic and electrophoresis phenomena, which enables the macromolecule to enter the nanochannel for molecular spreading.

According to the invention, the nanochannel is arranged on at least one nanofluidic chip. Advantageously, the nanofluidic chip comprises at least one array of nanochannels which enables the simultaneous manipulation of various macromolecules. According to the invention the expression "nanochannel array" designates an ensemble of at least two, non connected, nanochannels.

The nanofluidic chip may be fabricated from materials such as silicon, glass or polymers, especially PDMS (polydimethylsiloxane) by methods well known by the skilled person. Advantageously, nanofluidic chips of PDMS or silicon are obtained by PDMS-base shift lithography (PPSL), by electron microscopy, by projection lithography, or by nano-imprint lithography.

Once spreaded in the nanochannels, the macromolecules may be analysed as usual, and advantageously in continuous flow to maintain a constant elongation for the macromolecules conveyed in nanochannels. Advantageously, this method for analysing macromolecules could be used for structural analysis of macromolecules, in particular for genomic analysis of biological macromolecules. Especially, this method for analysing macromolecule could be used for the diagnosis of diseases such as cancer. Different types of analysis, including (i) the mapping of replication defects by monitoring the distribution of replication forks in a genome; (ii) the analysis of epigenetic marks along individual chromosomes in normal vs. cancer cells; (iii) the genomic analysis of linearized DNA for high throughput sequencing can be implemented.

The invention also relates to a device for carrying out the process described above, comprising:

- at least one nanochannel, the inlet of which is connected to at least one microfluidic channel; the nanochannel(s) may have the features described above with respect to the method; preferably the microfluidic channel is of 2 to 10 $\mu$m in height and of 10 to 100 $\mu$m in width;
- the microfluidic channel is connected to a feeding reservoir intended to comprise the fluid and the macromolecule;
- the feeding reservoir is connected to at least one mean for applying a controlled pressure to the fluid.

Preferably, the outlet of the nanochannel is connected to at least one microfluidic channel preferably of 2 to 10 $\mu$m in height and of 10 to 100 $\mu$m in width; this microfluidic channel is connected to a recovering reservoir to recover the fluid and the spreaded macromolecule. This recovering reservoir may be connected to the air (atmospheric pressure) or to another reservoir at a pre-determined or controlled pressure in order to control the pressure at the outlet.

In an embodiment, the mean for applying a controlled pressure to the fluid comprise an actuation reservoir comprising a liquid as actuation means. In this embodiment, there is a solid interface between the feeding reservoir and the actuation reservoir, such as a piston, which separates both liquids and which allows the pressure of the actuation liquid to be transmitted to the liquid in the feeding reservoir. Advantageously, both reservoirs are of similar geometry with the piston separating both reservoirs. In another embodiment, the actuation fluid is a gas. The same structure may be used. It is also possible not to include the solid interface, as soon as the gas may transmit the pressure without mixture with the liquid in the feeding reservoir, i.e. the gas may be in direct contact with the fluid. Suitable means of this type are described in WO2004/103566.

In an embodiment, when electric field is generated in addition to the hydrostatic pressure, the device comprises platinum electrodes which are placed into the feeding and recovering reservoir and are intended to be in contact with the fluid in order to impose the electrical potential to the fluid from the inlet to the outlet, as this is well known to the person skilled in the art.

In order to analyse the linear obtained macromolecule, the device may comprise or be connected to, e.g. at the outlet of the nanochannel, suitable means for the intended analysis.

Preferably, the device comprises:

- a nanochannel array, the inlet and outlet of which are connected to two independent microfluidic channels, preferably of 2-10 $\mu$m in height and 10-100 $\mu$m in width;
- the microfluidic channel connected to the inlet of the nanochannels is connected to a feeding reservoir intended to comprise the fluid and the macromolecules;
- the feeding reservoir is connected to means for applying a controlled pressure to the fluid;
- the microfluidic channel connected to the outlet of the nanochannels is connected to a recovering reservoir to recover the fluid and the spreaded macromolecules and this recovering reservoir is connected to air or another reservoir at a pre-determined or controlled pressure.

The analysis device consists of an optical or electrical sensor located at the middle of the nanochannels. In the case of optical sensors, the device comprises a high-magnification objective connected to a video camera, which records the passage of the macromolecules over time. Instead of using the full matrix of a camera, the recording device can consist of a linear array of sensors that measure a light signal associated to the passage of macromolecules in individual nanochannels, having precisely measured the velocity of the said macromolecules. Alternatively an array of electrodes may be placed midway through the nanochannels in order to sense the passage of macromolecules.

In an embodiment, the inlet microfluidic channel has two extremities, each one of them being connected to a feeding reservoir, which is itself connected to its own actuating reservoir. At the center of the microfluidic channel, there is an area where the nanochannels are connected. A similar structure may be used for the outlet, with an outlet microfluidic channel having two extremities, each one of them being connected to a recovering reservoir.

The invention will now be defined with non limitative examples and by reference to the drawings. When the dimensions of the nanochannels are given it should be understood W*H or WxH.

Figure 1 represents a nanochannel presenting a circular cross-section according to the invention with a diameter D and a length L.

Figure 2 represents a nanochannel presenting a square or rectangular cross-section according to the invention with a larger diagonal LD, a width W, a height H and a length L.

Figure 3 represents a cross-sectional view of a nanochannel according to the invention, illustrating the parabolic profile of the flow rate v(y).

Figure 4 represents a microscopic view of the entry of lambda phage DNA molecule by electrophoresis and by the process according to the invention in a nanochannel.

Figure 5 represents different positions of the macromolecule in the nanochannel.

Figure 6 represents the evolution of the spreading (or stretching) (in $\mu$m) of lambda phage DNA according to the molecule velocity.

Figure 7 represents the relaxation of lambda DNA inside nanochannels.

Figure 8 represents a device according to the present invention.

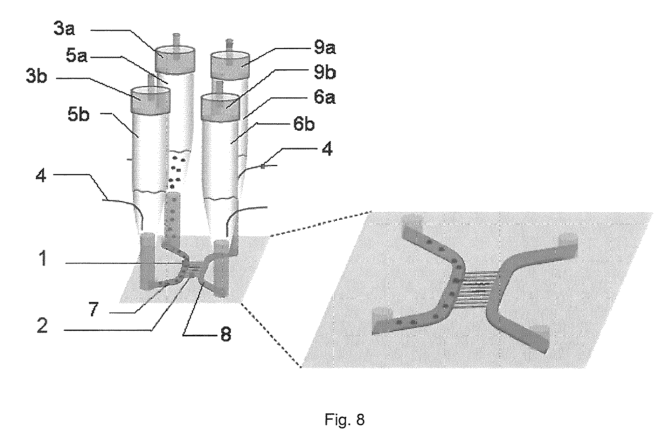

On figure 8, the device comprises:

- a nanochannel array 2 with the inlet and outlet connected to independent inlet and outlet microfluidic channels, respectively 7 and 8; the dimensions of these channels is of 2-10 $\mu$m microns in height and 10-100 $\mu$m in width;
- the inlet microfluidic channel 7 has a form of a U, with the basis of the U being connected to the inlet of the nanochannels 1 (see the enlarged part on figure 8); at each of its extremities the channel 7 is connected to a feeding reservoir 5a, 5b intended to comprise the fluid and the macromolecules;
- both feeding reservoirs 5a and 5b are connected to means 3a and 3b for applying a controlled pressure to the fluid;
- the outlet microfluidic channel 8 has a form of a U, with the basis of the U being connected to the outlet of the nanochannels 1 (see the enlarged part on figure 8); at each of its extremities the channel 8 is connected to a recovering reservoir 6a, 6b intended to recover the fluid and the macromolecules passing through the nanochannels;
- both recovering reservoirs 6a and 6b are connected to a supplemental reservoir 9a, respectively 9b, connected to the air or which internal pressure is controlled.

In one embodiment, when electric field is generated in addition to the hydrostatic pressure, the device comprises platinum eletrodes 4 which are placed into the feeding and recovering reservoir and are intended to be in contact with the fluid.

Example 1 : Influence of the hydrodynamic pressure on the conformation of the molecule (comparative example of spreading by the process according to the invention or by electrophoresis)

This experiment was implemented by time-series fluorescence microscopy of a single DNA molecule entering into a nanochannel using electrophoretic or hydrodynamic actuation (upper and lower panel, respectively).

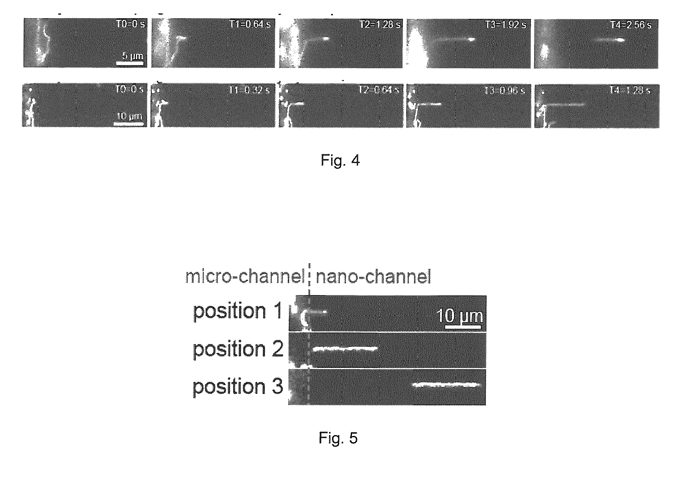

The results are shown in figure 4.

$\lambda$-DNA (50 10$^3$ base pairs) stained with the DNA intercalating fluorophore YOYO-1 (excitation/emission wavelength 488/510 nm) was observed by fluorescence microscopy using a LED light source emitting at 475+/-28 nm (10 mW) and an Electron-Multiplied CCD camera.

The device used for carrying out the experiment is composed of 200 nmX200 nm PDMS nanochannels of 100 $\mu$m in length. In the upper panel, the electric field is set to +40 V in the inlet microchannel (left side of the image), and the outlet microchannel is connected to the ground. In the lower panel, the pressures in the inlet and outlet microchannels are set to +400 and +10 mBar, respectively.

In both cases, the molecules initially adopts a hairpin configuration, which remains stable during at least 2 s with electrophoresis, as inferred from the enhanced fluorescence signal at the leading edge of the molecule. In contrast, the hairpin is rapidly unfolded using hydrodynamic actuation, as shown by the relaxation into a linear and homogeneously stained state. These hydrodynamic-specific relaxations of hairpins are accounted for by the slowed down intrinsic dynamics of DNA molecules in the presence of Poiseuille flows. Slow dynamics imply that the molecule is characterized by an enhanced deformability for a given flow rate (see e.g. F. Brochard-Wyart 1995 Europhys. Lett. 30 387 "Polymer Chains Under Strong Flows: Stems and Flowers").

The conformation of $\lambda$-DNA molecules, which were imaged by fluorescence microscopy in the same conditions as in figure 4, was analyzed by image analysis at their uptake in different types of nanochannels. The conformation of a molecule was considered as linear, when we could not detect any accumulation of fluorescence at its leading edge (see upper panel in Fig. 4). We then evaluated the average number of linear molecule based on the conformation of 30 to 40 independent molecules. The migration speed of the DNA molecules was set to 100+/-20 $\mu$m/s in every experiment.

The table below represents the percentage of molecules in an elongated conformation for different nanofluidic devices and using hydrodynamic or electrophoretic actuation. The percentages are measured at three different positions, which are represented in figure 5. Optimal results, for which 95% of the molecules adopt a linear conformation, are reached using 200 nmx200 nm PDMS (polydimethylsiloxane) nanochannels.

|   | Method | Device | pos 1 | pos 2 | pos 3 |
|---|---|---|---|---|---|
| 1 | | Si 150nmx200nmx100 $\mu$m | 49% | 49% | 49% |
| 2 | | Si 300 nmx250 nmx100 $\mu$m | 69% | 72% | 72% |
| 3 | Hydrodynamics | Si 500 nmx250 nmx500 $\mu$m | 37% | 37% | 37% |
| 4 | | PDMS with distortion 200 nmx200 nmx100$\mu$m | 50% | 82% | 95% |
| 5 | | PDMS without distortion 200 nmx200 nmx500 $\mu$m | 66% | 89% | 89% |
| 6 | Electrophoresis | PDMS with distortion 200 nmx200 nmx100 $\mu$m | 17% | 17% | 17% |

Channels containing a distorsion bear a constriction at a distance of ~10 $\mu$m from the inlet (see electron micrograph of Fig. 1 sent in May). The stress exerted by the shear flow is enhanced in the constriction, leading to an enhanced transient elongation of ~ 19 $\mu$m (see histograms of Fig. 2 sent in May), and to an improved relaxations of hairpin conformations, as shown in this table. Note that two PDMS devices, the first one bearing a constriction, and the other consisting of straight nanochannels, were fabricated.

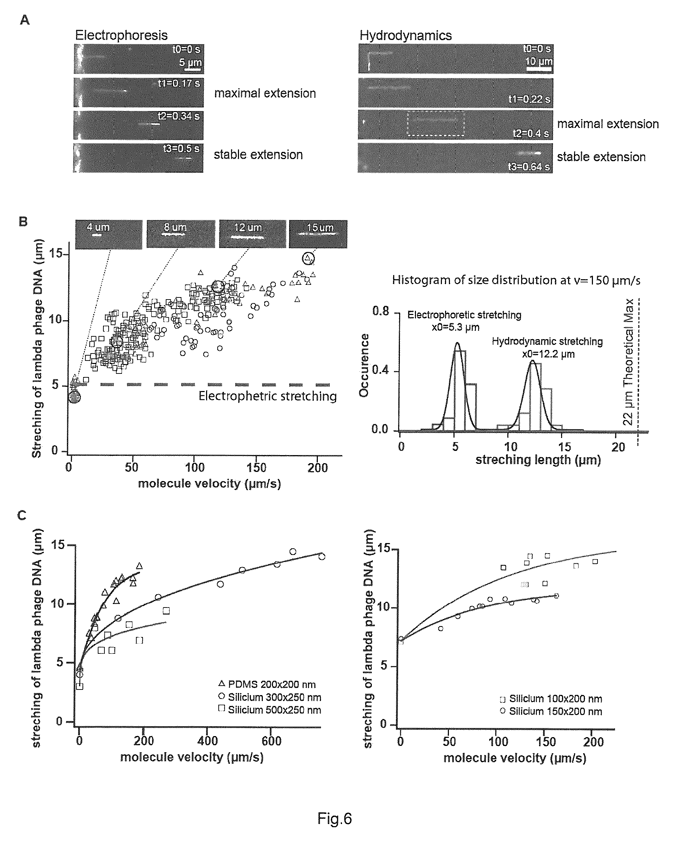

Example 2: Influence of the nanochannel on the efficiency and the stretching length (figure 6)

The experiment relates to time-series fluorescence microscopy of linearly-elongated $\lambda$-DNA molecules entering into nanochannels using electrophoretic or hydrodynamic actuation (left and right lanes, respectively). For figure 6A, the conformational dynamics was studied by real time fluorescence microscopy using the device described in example 1 for figure 4 (200 nmX200 nm square PDMS nanochannels of 100 $\mu$m in length). The length of $\lambda$-DNA molecules was measured using the imageJ freeware (rsbweb.nih.gov/ij/) and the default threshold macro to segment the molecules, followed by length measurements based on ellipse fitting (these two functions are in the freeware).

Figure 6A shows that the maximal elongation is reached right after the uptake inside the nanochannel, and the molecule then relaxes into a steady conformation that is measured in the case of hydrodynamics and electrophoresis.

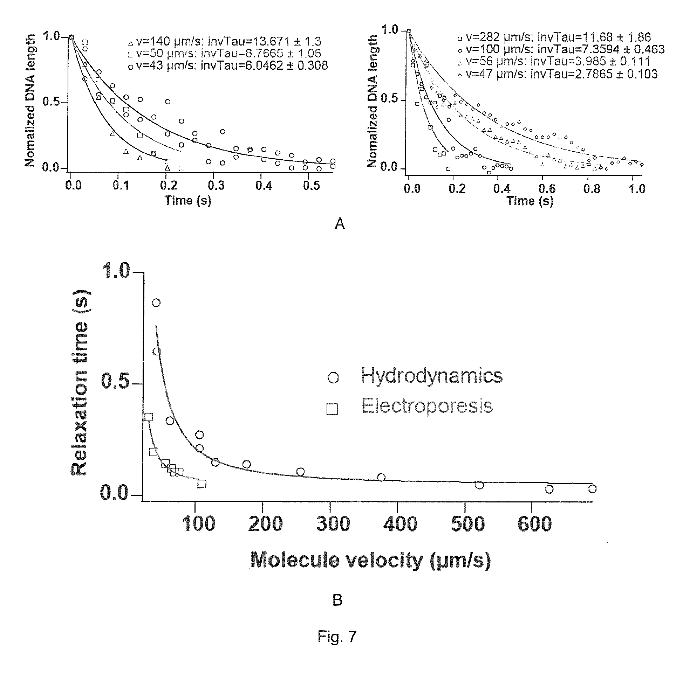

For figure 6B, the stable extension of the molecule (that is the constant elongation reached after a step of relaxation described in Fig. 7B) is recorded for different DNA migration velocities, which are obtained by adjusting the pressure drop between the inlet and the outlet channels in the range +80 to +1000 mBar.

The migration velocity of electrophoretic-driven molecules was adjusted by varying the potential in the inlet channel from +20 to +200 V (the potential of the outlet channel being at the ground). The stable extension was similarly measured, showing no elongation with electric actuation (green dashed line).

In figure 6B, the left plot represents the elongation of the molecule as a function of its migration speed, as recorded by temporal evolution of the center of mass of the molecule. The upper insert shows four fluorescence micrographs of molecules of different steady elongation. The histogram on the right shows the elongation of 15 molecules conveyed at 150 $\mu$m/s using hydrodynamic or electrophoretic actuation. We note an enhancement of the stretching of 150%.

For figure 6C, the stable extension was measured for different DNA migration velocities, as described in (6B), and for different nanochannels geometries: PDMS 200*200nm, silicium 300*250nm, silicium 500*250 nm, silicium 100*200 nm, silicium 150*200nm. Each data point corresponds to the average of at least 15 molecules.

Example 3: Relaxation of λ-DNA molecules inside the nanochannels (figure 7)

The length of single λ-DNA molecules entering in nanochannels was recorded over time using the protocol described for Fig. 6A. The data was offset by the stable elongation, and then normalized to the maximal extension. The normalized data was fitted with a single exponential relaxation model (curves appended to the datasets). This work was carried out in two PDMS and silicon nanochannels (left and right plots, respectively) of different geometries, as indicated in the inset. The migration velocity was tuned by adjusting the pressure in the inlet channel between +100 to +500 mBar.

In figure 7A, the length of individual DNA molecules is recorded over time starting from the maximal extension (t=0 s, see time series in Fig. 6A wherein the time for the maximal extension for electrophoresis is 0.17 s and the time for the maximal extension for hydrodynamics is 0.4 s), and then plotted with a normalized length. We note that the relaxation time, as inferred from the exponential fitting of the relaxation curves, decreases as the DNA migration speed increases.

The relaxation time is plotted as a function of the migration speed, each data point represents an average over at least 5 molecules. The experiments was carried out using electrophoresis to convey DNA in nanochannels (this experiment has been carried out using the protocol described in Fig. 6B).

In figure 7B, the relaxation timescale is represented as a function of DNA migration speed using hydrodynamic (circles) and electrophoretic (squares) actuation. We note that the relaxation time is systematically longer with hydrodynamics, showing that the intrinsic dynamics of DNA is altered by the presence of Poiseuille flows. This slowed down dynamics suggests that the deformability of DNA is higher with hydrodynamics, and accounts for the relaxation of hairpin configurations at the uptake of molecules (Fig. 4).

## Claims

1. Method for longitudinal macromolecule spreading by passing a fluid comprising said macromolecule in a nanochannel by applying to this fluid a hydrostatic pressure.

2. Method according to claim 1, wherein the nanochannel has a section having a larger diagonal (or diameter) comprised between 100 and 600 nm, and a length (L) greater or equal to 20 $\mu$m, preferably comprised between 100 and 1000 $\mu$m.

3. Method according to claim 1, wherein the nanochannel has a rectangular, square, oval or circular section having a larger diagonal (or diameter) comprised between 100 and 600 nm, and a length (L) greater or equal to 20 $\mu$m, preferably comprised between 100 and 1000 $\mu$m.

4. Method according to claim 1, wherein the nanochannel has a rectangular, square, oval or circular section having a larger diagonal (or diameter) comprised between between 200 and 300 nm, and a length (L) greater or equal to 20 $\mu$m, preferably comprised between 100 and 1000 $\mu$m.

5. Method according to claim 1, wherein the nanochannel has a rectangular or square section having :

    - a width (W) comprised between 100 and 500 nm;
    - a height (H) comprised between 100 and 500 nm; and
    - a length (L) comprised between 100 and 200 $\mu$m, preferably between 100 and 150 $\mu$m.

6. Method according to claim 1, wherein the nanochannel has a rectangular or square section having :

    - a width (W) comprised between 200 and 300 nm;
    - a height (H) comprised between 200 and 300 nm; and
    - a length (L) comprised between 100 and 200 $\mu$m, preferably between 100 and 150 $\mu$m.

7. Method according to anyone of claims 1 to 6, wherein the hydrostatic pressure applied to the fluid at the inlet of the nanochannel is less than 1 MPa, preferably less than 0.2 MPa, for example comprised between 0.01 and 0.1 MPa.

8. Method according to anyone of claims 1 to 7, wherein the pressure at the outlet of the nanochannel is comprised between 0.001 and 0.005 MPa, in particular comprised between 0.001 and 0.002 MPa, for example the pressure at the outlet of the nanochannel is of 0.001 MPa.

9. Method according to anyone of claims 1 to 8, wherein the flow rate is comprised between 10 et 10000 $\mu$m/s, preferably between 100 and 1000 $\mu$m/s.

10. Method according to anyone of claims 1 to 9, wherein

   - for a length comprised between 50 and 200$\mu$m, and a larger diagonal (or diameter) comprised between 200 and 300 nm, the pressure drop between the inlet and outlet channels is comprised between 0.002 and 0.016 MPa for a mean flow velocity of 100 $\mu$m/s ; or
   - for a length comprised between 50 and 200 $\mu$m and a larger diagonal (or diameter) comprised between 200 and 300 nm, the pressure drop is comprised between 0.02 and 0.16 MPa for a mean flow velocity of 1000 $\mu$m/s.

11. Method according to anyone of claims 1 to 9, wherein

   - for a larger diagonal (or diameter) comprised between 200 and 300 nm, the mean flow rate (v) is comprised between 50 and 500 $\mu$m/s; or
   - for a larger diagonal (or diameter) comprised between 200 and 400 nm the flow rate (v) is comprised between 100 and 500 $\mu$m/s; or
   - for a larger diagonal (or diameter) comprised between 100 nm and 300 nm, the flow rate (v) is comprised between 50 and 200 $\mu$m/s.

12. Method according to anyone of claims 1 to 11, wherein the fluid comprising the macromolecule is a buffer solution of physiological pH between 5 and 9, more preferably between 7 and 8.

13. Method according to anyone of claims 1 to 12, wherein the salt concentration in the fluid is comprised between 1 and 500 mM, more preferably between 20 and 200 mM.

14. Method according to anyone of claims 1 to 13, further comprising the implementation of an electric field.

15. A device for carrying out the method of claims 1 to 14, which comprises

   - at least one nanochannel, the inlet of which is connected to at least one microfluidic channel preferably of 2 to 10 $\mu$m in height and of 10 to 100 $\mu$m in width;
   - the microfluidic channel is connected to a feeding reservoir intented to comprise the fluid and the macromolecule;
   - the feeding reservoir is connected to at least one mean for applying a controlled pressure to the fluid.

16. A device according to claim 15 which further comprises at the outlet of the nanochannel, a suitable mean for the intended analysis.

17. Device according to claim 15 or 16, wherein the mean for applying a controlled pressure to the fluid comprises an actuation reservoir comprising a liquid or a gas.

Fig.1

Fig.2

Fig.3

Fig. 4

Fig. 5

Fig.6

A

B

Fig. 7

Fig. 8

Europäisches
Patentamt

European
Patent Office

Office européen
des brevets

**EUROPEAN SEARCH REPORT**

Application Number

EP 11 30 6169

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KRISTIAN H. RASMUSSEN ET AL: "A device for extraction, manipulation and stretching of DNA from single human chromosomes", LAB ON A CHIP, vol. 11, no. 8, 1 January 2011 (2011-01-01), page 1431, XP55018852, ISSN: 1473-0197, DOI: 10.1039/c01c00603c * the whole document * | 1-17 | INV. C12Q1/68 B01L3/00 |
| X | US 2008/242556 A1 (CAO HAN [US] ET AL) 2 October 2008 (2008-10-02) * paragraph [0132] - paragraph [0133] * * paragraph [0142]; claims 61,166,167,184 * | 1-17 | |
| X | WO 01/13088 A1 (US GENOMICS INC [US]) 22 February 2001 (2001-02-22) * page 28 - page 40, line 10; claim 266 * | 1-17 | |
| A | WO 2010/042007 A1 (TEGENFELDT JONAS [SE]; REISNER WALTER [CA]; FLYVBJERG HENRIK [DK]) 15 April 2010 (2010-04-15) * page 14, line 26 - page 15, line 3 * | 10 | TECHNICAL FIELDS SEARCHED (IPC)  C12Q B01L |
| A | WO 2004/091795 A2 (US GENOMICS INC [US]; FUCHS MARTIN [US]; LARSON JONATHAN [US]) 28 October 2004 (2004-10-28) * page 35, line 5 - line 16 * | 10 | |

-/--

The present search report has been drawn up for all claims

| Place of search | Date of completion of the search | Examiner |
|---|---|---|
| The Hague | 9 February 2012 | Gabriels, Jan |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 11 30 6169

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | J. O. TEGENFELDT: "From the Cover: The dynamics of genomic-length DNA molecules in 100-nm channels", PROCEEDINGS OF THE NATIONAL ACADEMY OF SCIENCES, vol. 101, no. 30, 19 July 2004 (2004-07-19), pages 10979-10983, XP55018740, ISSN: 0027-8424, DOI: 10.1073/pnas.0403849101 * the whole document * | 1 | |
| | ----- | | |
| | | | **TECHNICAL FIELDS SEARCHED (IPC)** |

The present search report has been drawn up for all claims

| Place of search | Date of completion of the search | Examiner |
|---|---|---|
| The Hague | 9 February 2012 | Gabriels, Jan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.............................................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 11 30 6169

This annex lists the patent family members relating to the patent documents cited in the above-mentioned European search report.
The members are as contained in the European Patent Office EDP file on
The European Patent Office is in no way liable for these particulars which are merely given for the purpose of information.

09-02-2012

| Patent document cited in search report | | Publication date | Patent family member(s) | | Publication date |
|---|---|---|---|---|---|
| US 2008242556 | A1 | 02-10-2008 | AU 2008232616 A1 | | 09-10-2008 |
| | | | CA 2682275 A1 | | 09-10-2008 |
| | | | CN 101765462 A | | 30-06-2010 |
| | | | EP 2136922 A2 | | 30-12-2009 |
| | | | JP 2010522569 A | | 08-07-2010 |
| | | | KR 20100018495 A | | 17-02-2010 |
| | | | US 2008242556 A1 | | 02-10-2008 |
| | | | WO 2008121828 A2 | | 09-10-2008 |
| WO 0113088 | A1 | 22-02-2001 | AU 6771100 A | | 13-03-2001 |
| | | | CA 2381361 A1 | | 22-02-2001 |
| | | | CN 1379857 A | | 13-11-2002 |
| | | | EP 1210578 A1 | | 05-06-2002 |
| | | | JP 2003507026 A | | 25-02-2003 |
| | | | WO 0113088 A1 | | 22-02-2001 |
| WO 2010042007 | A1 | 15-04-2010 | US 2011201509 A1 | | 18-08-2011 |
| | | | WO 2010042007 A1 | | 15-04-2010 |
| WO 2004091795 | A2 | 28-10-2004 | EP 1620203 A2 | | 01-02-2006 |
| | | | JP 2006522940 A | | 05-10-2006 |
| | | | WO 2004091795 A2 | | 28-10-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 7670770 B
- US 7217562 B
- WO 2004103566 A

### Non-patent literature cited in the description

- **F. BROCHARD-WYART.** Polymer Chains Under Strong Flows: Stems and Flowers. *Europhys. Lett.,* 1995, vol. 30, 387